# FASCICULE DE BREVET EUROPEEN

(11) **EP 1 707 240 B1**
(45) Date de publication et mention de la délivrance du brevet: **28.04.2010**
(21) Numéro de dépôt: 06110784.3
(22) Date de dépôt: 07.03.2006
(51) Int. Cl.: A61Q 5/02, A61Q 19/10, A61K 8/37, A61K 8/39, A61K 8/44, A61K 8/46, A61K 8/73

(54) **Compositions cosmétiques détergentes comprenant trois tensioactifs et un ester gras et leurs utilisations**
Kosmetische Reinigungszusammensetzungen enthaltend drei Tenside und ein Fettsäureester sowie deren Verwendung
Detergent cosmetic compositions comprising three surfactants and a fatty ester, and use thereof

(30) Priorité: 30.03.2005 FR 0550808
(43) Date de publication de la demande: 04.10.2006
(73) Titulaire: L'Oréal, 75008 Paris (FR)
(72) Inventeur: Beauquey, Bernard, 92110 Clichy (FR); Maggio, Sandrine, 94300 Vincennes (FR); Meralli, Sabina, 92170 Vanves (FR)
(74) Mandataire: Le Blainvaux Bellegarde, Françoise

(56) Documents cités:
- EP-A- 1 034 775
- EP-A- 1 064 915
- EP-A- 1 232 739
- EP-A- 1 245 225
- EP-A- 1 250 906
- WO-A-00/72807
- DE-A1- 10 048 449
- DE-A1- 10 048 450
- DE-A1- 19 710 873
- US-A- 5 955 406
- US-A1- 2004 102 354
- US-B1- 6 284 230

## Description

La présente invention concerne de nouvelles compositions cosmétiques à propriétés améliorées destinées simultanément au nettoyage et au conditionnement des matières kératiniques telles que les cheveux, et comprenant, dans un support aqueux cosmétiquement acceptable, (A) au moins un tensioactif anionique sulfate ou sulfonate, (B) au moins un tensioactif anionique carboxylique différent du tensioactif cité en (A) choisi parmi les acides alkyl (C₆-C₂₄) éther carboxyliques polyoxyalkylénés, les acides alkyl(C₆-C₂₄)aryl éther carboxyliques polyoxyalkylénés et leurs sels, les acides alkyl(C₆-C₂₄) amido éther carboxyliques polyoxyalkylénés et leurs sels, (C) au moins un tensioactif amphotère, et (D) au moins un ester d'acide carboxylique insoluble dans l'eau lesdits esters sont choisis parmi :
1) les esters d'acide carboxylique en C3-C30 et d'alcool en C1-C30, l'un au moins de l'acide ou de l'alcool étant ramifié, et
2) les esters d'acide aromatique en C7-C30 dont la fonction carboxylique est directement liée au cycle aromatique et d'alcool en C1-C30,
la quantité d'esters allant de 0,5 à 10% en poids par rapport au poids total de la composition,
le rapport en poids tensioactif anionique sulfate ou sulfonate / tensioactif anionique carboxylique va de 2 à 12. L'invention concerne aussi l'utilisation desdites compositions dans l'application cosmétique susmentionnée.

Pour le nettoyage et/ou le lavage des matières kératiniques telles que les cheveux, l'utilisation de compositions détergentes (telles que les shampooings) à base essentiellement d'agents tensioactifs classiques de type notamment anionique, non ionique et/ou amphotère, mais plus particulièrement de type anionique, est courante. Ces compositions sont appliquées sur cheveux mouillés et la mousse générée par massage ou friction avec les mains permet, après rinçage à l'eau, l'élimination des diverses salissures initialement présentes sur les cheveux ou la peau.

Ces compositions de base possèdent certes un bon pouvoir lavant, mais les propriétés cosmétiques intrinsèques qui leur sont attachées restent toutefois assez faibles, notamment en raison du fait que le caractère relativement agressif d'un tel traitement de nettoyage peut entraîner à la longue sur la fibre capillaire des dommages plus ou moins marqués liés en particulier à l'élimination progressive des lipides ou protéines contenus dans ou à la surface de cette dernière.

Aussi, pour améliorer les propriétés cosmétiques des compositions détergentes ci-dessus, et plus particulièrement de celles qui sont appelées à être appliquées sur des cheveux sensibilisés (i.e. des cheveux qui se trouvent abîmés ou fragilisés notamment sous l'action chimique des agents atmosphériques et/ou de traitements capillaires tels que permanentes, teintures ou décolorations), il est maintenant usuel d'introduire dans ces dernières des agents cosmétiques complémentaires dits agents conditionneurs destinés principalement à réparer ou limiter les effets néfastes ou indésirables induits par les différents traitements ou agressions que subissent, de manière plus ou moins répétée, les fibres capillaires. Ces agents conditionneurs peuvent bien entendu également améliorer le comportement cosmétique des cheveux naturels.

Dans ce but, on a déjà proposé d'utiliser des agents conditionneurs insolubles. Ces composés insolubles présentent l'inconvénient d'être difficiles à maintenir en dispersion régulière dans le milieu.

Pour les maintenir en suspension, on a déjà proposé l'utilisation des dérivés d'esters ou d'éthers à longue chaîne (agents dispersants) ou des polysaccharides tels que la gomme de xanthane (agents gélifiants). Cependant, les agents dispersants présentent des problèmes de cristallisation qui entraînent parfois une évolution (augmentation) de la viscosité des compositions au cours du temps ; les agents gélifiants présentent également des inconvénients, à savoir d'une part que la mousse des compositions détergentes contenant des polysaccharides se développe difficilement (mauvais démarrage de mousse) et que, d'autre part, les compositions n'ont pas une texture lisse et s'écoulent par paquets, ce qui est peu apprécié des utilisateurs. De plus, ces divers agents ne permettent pas d'obtenir des compositions transparentes ou limpides.

US2004/0102354 décrit des compositions détergentes comprenant un tensioactif anionique sulfate, un tensioactif anionique carboxylique dans un rapport inférieur à 2, un tensioactif amphotère et un ester d'acide gras différent des esters de l'invention.

EP 1245225 décrit des compositions détergentes comprenant un tensioactif anionique sulfate, un tensioactif anionique carboxylique de type glutamate, un tensioactif amphotère et un ester d'acide gras.

EP 1034775 décrit des compositions détergentes comprenant un tensioactif anionique sulfate, un tensioactif amphotère et un ester d'acide gras.

DE19710873 décrit des compositions détergentes comprenant un tensioactif anionique sulfonate, un tensioactif anionique carboxylique dans un rapport inférieur à 2, un tensioactif amphotère et 0,3% d'un ester d'acide gras.

La présente invention a pour but de proposer des compositions ne présentant pas les inconvénients des compositions citées ci-dessus.

Les agents conditionneurs doivent également être véhiculés sur les matières kératiniques traitées en vue de leur conférer, suivant l'application, des propriétés de douceur, de brillance et de démêlage.

Ainsi, à la suite d'importantes recherches menées sur la question, il a maintenant été trouvé par la Demanderesse, qu'en utilisant une association de 3 types de tensioactifs et au moins un ester d'acide carboxylique, il est possible d'obtenir des compositions détergentes stables présentant d'excellentes propriétés cosmétiques, en particulier le démêlage et le lissage des cheveux traités et ayant de bonnes propriétés d'usage tel qu'un bon pouvoir lavant intrinsèque et un bon pouvoir moussant.

La mise en oeuvre industrielle est extrêmement facile et les propriétés cosmétiques des shampooings sont excellentes.

Les compositions obtenues sont stables au stockage, sans nécessiter l'addition d'agent de dispersion et/ou de mise en suspension de l'ester selon l'invention.

En l'absence de composés additionnels insolubles, les compositions obtenues sont également transparentes. Elles peuvent contenir des quantités importantes d'ester d'acide carboxylique tout en conservant une bonne transparence et en ayant de bonnes propriétés cosmétiques.
Les compositions conformes à l'invention présentent de très bonnes qualités d'usage (mousse abondante, aérée et se développant rapidement) ainsi qu'une très bonne rinçabilité.

Les compositions conformes à l'invention confèrent aux cheveux, notamment après rinçage, un remarquable effet traitant qui se manifeste notamment par une facilité de démêlage, ainsi qu'un apport de lissage, de douceur et de souplesse sans aucune sensation de gras. Le toucher est « naturel » et plus propre.
De plus, ces compositions apportent du coiffant sur cheveux séchés (apport de corps et de texture) rendent la mise en forme de la coiffure plus facile le jour de l'application sur les cheveux les plus difficiles à maîtriser. De plus cette coiffure tient mieux dans le temps.

Ainsi, la présente invention a pour objet de nouvelles compositions cosmétiques détergentes caractérisée par le fait qu'elles comprennent, dans un milieu aqueux cosmétiquement acceptable, (A) au moins un tensioactif anionique sulfate ou sulfonate, (B) au moins un tensioactif anionique carboxylique différent du tensioactif cité en (A) choisi parmi les acides alkyl (C₆-C₂₄) éther carboxyliques polyoxyalkylénés, les acides alkyl(C₆-C₂₄)aryl éther carboxyliques polyoxyalkylénés et leurs sels, les acides alkyl(C₆-C₂₄) amido éther carboxyliques polyoxyalkylénés et leurs sels, (C) au moins un tensioactif amphotère, et (D) au moins un ester d'acide carboxylique insoluble dans l'eau lesdits esters sont choisis parmi :
1) les esters d'acide carboxylique en C3-C30 et d'alcool en C1-C30, l'un au moins de l'acide ou de l'alcool étant ramifié, et
2) les esters d'acide aromatique en C7-C30 dont la fonction carboxylique est directement liée au cycle aromatique et d'alcool en C1-C30,
la quantité d'esters allant de 0,5 à 10% en poids par rapport au poids total de la composition,
le rapport en poids tensioactif anionique sulfate ou sulfonate / tensioactif anionique carboxylique va de 2 à 12.

L'invention a également pour objet l'utilisation en cosmétique des compositions ci-dessus pour le nettoyage et /ou le démaquillage et/ou le conditionnement des matières kératiniques telles que les cheveux et la peau.

### (A) Tensioactifs sulfate(s) ou sulfonate(s)

Selon l'invention, les tensioactifs anioniques sulfate(s) ou sulfonate(s) sont des tensioactifs anioniques comportant au moins une fonction sulfate (-OSO₃H ou - OSO₃-) et/ou une fonction sulfonate (-SO₃H ou -SO₃-).

Les tensioactifs anioniques sulfates ou sulfonates utilisables, seuls ou mélanges, dans le cadre de la présente invention, sont les sels (en particulier sels alcalins, notamment de sodium, sels d'ammonium, sels d'amines, sels d'aminoalcools ou sels de magnésium) des alkylsulfates, alkylamidosulfates, alkyléthersulfates, alkylamidoéthersulfates, alkylaryléthersulfates, des alkyléthersulfosuccinates, des acyl iséthionates, des méthyl acyl taurates; le radical alkyle ou acyle de tous ces différents composés comportant de préférence de 8 à 24 atomes de carbone, et le radical aryle désignant de préférence un groupement phényle ou benzyle.

Le nombre moyen de groupements oxyde d'éthylène ou oxyde de propylène peut aller notamment de 2 à 50 et plus particulièrement de 2 à 10.

Parmi ces tensioactifs anioniques, on préfère utiliser les sels d'alkyléthersulfates en C₈-C₁₄ et plus particulièrement ceux en C₁₂-C₁₄. Ces sels comprennent de préférence de 2 à 5 groupements d'oxyde d'éthylène.
On utilise de préférence un agent tensioactif anionique choisi parmi les alkyl(C₁₂-C₁₄) sulfates de sodium, de triéthanolamine, de magnésium ou d'ammonium, les alkyl (C₁₂-C₁₄)éthersulfates de sodium, d'ammonium ou de magnésium oxyéthylénés à 2,2 moles d'oxyde d'éthylène, le cocoyl iséthionate de sodium et les méthyl acyl taurate.

Les tensioactifs anioniques sulfates ou sulfonates sont généralement présents à raison de 1,5 % à 50 % en poids, de préférence de 2 à 25 % en poids, particulièrement de 5 à 25% en poids et plus particulièrement de 8 à 20% en poids, et encore plus préférentiellement de 10 à 16% en poids par rapport au poids total de la composition.

### (B) Tensioactifs anioniques carboxyliques

Selon l'invention, les tensioactifs anioniques carboxyliques sont des tensioactifs anioniques comportant au moins une fonction carboxylique (-COOH) éventuellement sous forme de sel (-COO-).

Les tensioactifs anioniques de type carboxyliques différents des tensioactifs (A) ne comprennent de préférence pas de fonction sulfate ou sulfonate et sont choisis parmi les acides alkyl (C₆-C₂₄) éther carboxyliques polyoxyalkylénés, les acides alkyl(C₆-C₂₄)aryl éther carboxyliques polyoxyalkylénés et leurs sels, les acides alkyl(C₆-C₂₄) amido éther carboxyliques polyoxyalkylénés et leurs sels, en particulier ceux comportant de 2 à 50 groupements oxyde d'alkylène en particulier d'éthylène tels que les composés proposés par la société KAO sous les dénominations AKYPO.
On peut également utiliser les mélanges de ces tensioactifs.

Les sels sont en particulier choisis parmi les sels alcalins, notamment de sodium, les sels d'ammonium, les sels d'amines, les sels d'aminoalcools tels la triéthanolamine ou la monoéthanolamine et les sels de magnésium.

On utilise de préférence les acides alkyl (C₆-C₂₄) éther carboxyliques polyoxyalkylénés et leurs sels, les acides alkyl(C₆-C₂₄) amido éther carboxyliques polyoxyalkylénés en particulier ceux comportant de 2 à 15 groupements oxyde d'alkylène, et leurs sels et leurs mélanges.

Les tensioactifs anioniques du type acides ou sels d'éthers carboxyliques polyoxyalkylénés sont en particulier ceux qui répondent à la formule (1) suivante : 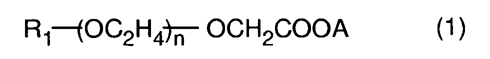 dans laquelle :
R1 représente un radical ou un mélange de radicaux alkyle ou alcényle linéaire ou ramifié en C₈-C₂₂, un radical alkyl(C₈-C₉)phényle, un radical R2CONH-CH₂-CH₂- avec R2 désignant un radical alkyle ou alcényle linéaire ou ramifié en C₁₁-C₂₁,
et n est un nombre entier ou décimal (valeur moyenne) pouvant varier de 2 à 24 et de préférence de 2 à 10, le radical alkyle ayant entre 6 et 20 atomes de carbone environ, de préférence de 8 à 18 atomes de carbone et aryle désignant de préférence phényle,

A désigne H, ammonium, Na, K, Li, Mg ou un reste monoéthanolamine ou triéthanolamine. On peut également utiliser des mélanges de composés de formule (1) en particulier des mélanges dans lesquels les groupements R₁ sont différents.

Les acides éther carboxylique oxyalkylénés ou leurs sels utilisés de préférence selon la présente invention sont choisis parmi ceux de formule (I) dans laquelle R1 désigne un radical ou un mélange de radicaux alkyl(C₁₂-C₁₄), cocoyle, oléyle; un radical nonylphényle ou octylphényle, A désigne un atome d'hydrogène ou de sodium, et n varie de 2 à 20 et de préférence 2 à 10.

Plus préférentiellement encore, on utilise des composés de formule (I) dans laquelle R désigne un radical alkyl(C₁₂), A désigne un atome d'hydrogène ou de sodium, et n varie de 2 à 10.

Parmi les produits commerciaux, on peut utiliser de préférence les produits vendus par la société CHEM Y sous les dénominations :
AKYPO® NP 70 (R=nonylphényle, n=7, p=0, A=H)
AKYPO® NP 40 (R=nonylphényle, n=4, p=0, A=H)
AKYPO® OP 40 (R=octylphényle, n=4, p=0, A=H)
AKYPO® OP 80 (R=octylphényle, n=8, p=0, A=H)
AKYPO® OP 190 (R=octylphényle, n=19, p=0, A=H)
AKYPO® RLM 38 (R= alkyl(C₁₂-C₁₄), n=3,8, p=0, A=H)
AKYPO® RLM 38 NV (R= alkyl(C₁₂-C₁₄), n=4, p=0, A=Na)
AKYPO® RLM 45 (R= alkyl(C₁₂-C₁₄), n=4,5, p=0, A=H)
AKYPO® RLM 45 NV (R= alkyl(C₁₂-C₁₄), n=4,5, p=0, A=Na)
AKYPO® RLM 100 (R= alkyl(C₁₂-C₁₄), n=10, p=0, A=H)
AKYPO® RLM 100 NV (R= alkyl(C₁₂-C₁₄), n=10, p=0, A=Na)
AKYPO® RLM 130 (R= alkyl(C₁₂-C₁₄), n=13, p=0, A=H)
AKYPO® RLM 160 NV (R= alkyl(C₁₂-C₁₄), n=16, p=0, A=Na)
ou par la société SANDOZ sous les dénominations :
SANDOPAN DTC-Acid (R= alkyl(C₁₃), n=6, p=0, A=H)
SANDOPAN DTC (R= alkyl(C₁₃), n=6, p=0, A=Na)
SANDOPAN LS 24 (R= alkyl(C₁₂-C₁₄), n=12, p=0, A=Na)
SANDOPAN JA 36 (R= alkyl(C₁₃), n=18, p=0, A=H),
et plus particulièrement, les produits vendus sous les dénominations suivantes : AKYPO® RLM 45
AKYPO® RLM 100
AKYPO® RLM 38.

Les tensioactifs anioniques carboxyliques différents des tensioactifs cités en A) sont généralement présents à raison de 0,5 % à 15 % en poids, de préférence de 1 à 10 % en poids, plus particulièrement de 1 à 5% en poids et encore plus préférentiellement de 1,5 à 3% en poids par rapport au poids total de la composition.

### (C) Tensioactif(s) amphotère(s) et/ou zwittérioniques:

Les agents tensioactifs amphotères et/ou zwittérioniques, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) bétaïnes ou les alkyl (C₈-C₂₀) amidoalkyl (C₁-C₆) sulfobétaïnes.

Parmi les dérivés d'amines, on peut citer les produits décrits dans les brevets US-2 528 378 et US-2 781 354 et de structures :

R₂ -CONHCH₂CH₂ -N(R₃)(R₄)(CH₂COO-) (2)

dans laquelle : R₂ CO désigne un radical acyle en C6-C24, par exemple un radical présent dans l'huile de coprah hydrolysée, un radical octoyle, décoyle ou dodécanoyle et leurs mélanges, R₃ désigne un groupement bêta-hydroxyéthyle et R₄ un groupement carboxyméthyle ;
et

R₂-CONHCH₂CH₂-N(B)(C) (3)

dans laquelle :
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂ - CHOH - SO₃H
R'₂ CO désigne un radical acyle en C6-C24, par exemple un radical présent dans l'huile de coprah hydrolysée ou l'huile de lin, un radical octoyle, décoyle ou dodécanoyle, stéaroyle ou isostéaroyle, oléoyle et leurs mélanges.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.
A titre d'exemple on peut citer le disodium cocoamphodiacetate commercialisé sous la dénomination commerciale MIRANOL^{®} C2M concentré par la société RHODIA CHIMIE.

Selon la présente invention, on préfère plus particulièrement utiliser les agents tensio-actifs amphotères appartenant au groupe des bétaïnes tels que les alkylbétaïnes en particulier la cocoylbétaïne commercialisée sous la dénomination « DEHYTON AB 30 » en solution aqueuse à 30 % de MA par la société HENKEL ou les alkylamidobétaines, en particulier la cocamidopropylbétaine telles que la TEGOBETAINE^{®} F50 commercialisée par la société GOLDSCHMIDT.

Le ou les tensio-actif(s) amphotère(s) sont généralement présents dans des quantités allant de 0,1 à 20 % en poids, de préférence de 1 à 15 % en poids, et plus particulièrement de 1,5 à 5% en poids par rapport au poids total de la composition.

La quantité minimale de tensioactifs est celle suffisante pour conférer à la composition finale un pouvoir moussant et/ou détergent satisfaisant, et des quantités trop importantes de base lavante n'apportent pas vraiment d'avantages supplémentaires.

Ainsi, selon l'invention, la quantité totale de tensioactifs peut représenter de 4 % à 50 % en poids, de préférence de 6 % à 35 % en poids, et encore plus préférentiellement de 8 % à 25 % en poids, et encore plus particulièrement de 14 à 20 % en poids du poids total de la composition finale.

Le rapport en poids tensioactif anionique sulfate ou sulfonate / tensioactif amphotère va de préférence de 2 à 12, plus particulièrement de 4 à 10 et encore plus préférentiellement de 5 à 8.

Le rapport en poids tensioactif anionique sulfate ou sulfonate / tensioactif anionique carboxylique va de préférence de 4 à 10 et plus particulièrement de 5 à 8.

Le rapport en poids tensioactif anionique carboxylique/ tensioactif amphotère va de préférence de 0,3 à 3 et encore plus préférentiellement de 0,5 à 1,5.

### D- Esters d'acide carboxylique

Les esters d'acide carboxylique insolubles dans l'eau selon l'invention sont insolubles dans l'eau à une concentration supérieure ou égale à 0,1% en poids dans l'eau à 25°C, c'est à dire qu'ils ne forment pas dans ces conditions une solution isotrope transparente à l'oeil nu.

Les esters utilisables dans la présente invention sont de préférence monomériques. Ils sont de préférence non ioniques, non siliconés .

Les esters d'acide carboxylique insolubles dans l'eau selon l'invention peuvent contenir des groupements hydroxyles.

Le nombre total de carbone des esters de l'invention va de préférence de 12 à 50 et plus particulièrement de 16 à 40 et encore mieux de 16 à 30.

Selon l'invention, les esters utilisés sont de préférence liquides à température ambiante (25°C) et à pression atmosphérique (1 atm).

Les esters carboxyliques sont de préférence choisis parmi :
1) les esters d'acide carboxylique en C3-C30 et d'alcool en C1-C30, l'un au moins de l'acide ou de l'alcool étant ramifié ou possédant au moins une double-liaison carbone-carbone, et
2) les esters d'acide aromatique en C7-C30 dont la fonction carboxylique est directement liée au cycle aromatique et d'alcool en C1-C30,

Les esters d'acide carboxylique en C3-C30 et d'alcool en C1-C30, l'un au moins de l'acide ou de l'alcool étant ramifié ou insaturé, sont notamment choisis parmi les esters d'acide carboxylique en C6-C24 et d'alcool en C3-C20.

Les esters selon l'invention sont notamment choisis parmi :
les esters d'acide carboxylique ramifié ayant de 4 à 6 atomes de carbone et d'alcool ayant de 8 à 26 atomes de carbone,
les esters d'acide carboxylique linéaire ayant de 12 à 26 atomes de carbone et d'alcool ramifié ayant de 3 à 12 atomes de carbone,
les esters d'acide carboxylique linéaire ayant de 2 à 12 atomes de carbone et d'alcool ramifié ayant de 8 à 26 atomes de carbone.
les esters d'acide carboxylique ramifié ayant de 8 à 26 atomes de carbone, de préférence 8 à 12 et d'alcool ramifié ayant de 8 à 26 atomes de carbone, de préférence 8 à 12.

On peut citer le béhénate d'octyldodécyle ; le béhénate d'isocétyle ; le lactate d'isocétyle ; le lactate d'isostéaryle ; l'octanoate de isostéaryle ; l'octanoate d'isocétyle ; l'isostéarate d'isocétyle ; le laurate d'isocétyle ; le stéarate d'isocétyle ; l'octanoate d'isodécyle ; l'oléate d'isodécyle ; l'isononanoate d'isononyle ; le palmitate d'isostéaryle ; l'isostéarate de myristyle ; l'isononanoate d'octyle ; l'isononate de 2-éthylhexyle; l'isostéarate d'octyle ; l'érucate d'octyldodécyle ; le palmitate d'isopropyle, le palmitate d'éthyl-2-héxyle, le palmitate de 2-octyldécyle, les myristates d'alkyles ramifiés tels que le myristate d'isopropyle, de t-butyle, de 2-octyldodécyle, l'isostéarate d'hexyle, l'isostéarate de butyle, le stéarate d'isobutyle ; le laurate de 2-hexyldécyle.
De préférence, l'acide et l'alcool de l'ester sont saturés.

On peut également utiliser les esters liquides d'acide carboxylique ramifié ayant de 4 à 6 atomes de carbone et d'alcool ayant de 8 à 26 atomes de carbone.

Ces esters liquides ramifiés selon l'invention ont de préférence la formule suivante

R₁ COOR₂ (I)

dans laquelle :
R₁ désigne un radical hydrocarboné, linéaire ou ramifié, éventuellement mono ou polyhydroxylé, ayant de 3 à 5 atomes de carbone,
R₂ désigne un radical hydrocarboné, linéaire ou ramifié, éventuellement mono ou polyhydroxylé, ayant de 12 à 26 atomes de carbone, de préférence ayant de 16 à 22 atomes de carbone,

R₁ désigne de préférence un radical alkyle ramifié ayant de 3 à 5 atomes de carbone, et plus particulièrement un radical tertio-butyle.
R₂ désigne de préférence un radical alkyle saturé ou insaturé ayant 12 à 26 atomes de carbone, plus particulièrement ramifié et encore plus particulièrement choisi parmi les radicaux tridécyle, isocétyle, isostéaryle, octyldodécyle et isoarachidyle.

Les esters liquides ramifiés particulièrement préférés sont le néopentanoate d'isostéaryle (formule (I) dans laquelle R₁=tertio-butyle et R₂=isostéaryle), le néopentanoate de tridécyle, le néopentanoate d'isocétyle, et le néopentanoate d'isoarachidyle.

Les esters d'acide aromatique en C7-C30 et d'alcool en C1-C30 sont de préférence des esters d'acide aromatique en C7-C17 et d'alcool en C1-C20. Ces esters sont notamment les benzoates d'alkyle en C12-C15, le benzoate d'isostéaryle, le benzoate d'octyledodécyle, le benzoate de béhényle, le benzoate d'éthyl-2hexyle.

Les esters d'acide carboxyliques sont plus particulièrement choisis parmi :
le lactate d'isostéaryle ; le lactate de lauryle ; l'octanoate d'isostéaryle ; l'octanoate d'isocétyle ; l'octanoate d'isodécyle ;
l'isononanoate d'isononyle ; l'isononanoate d'octyle ; l'isononate de 2-éthylhexyle ; l'érucate d'octyldodécyle,
le palmitate d'isopropyle, le palmitate d'éthyl-2-hexyle,
le myristate d'isopropyle, le stéarate d'isobutyle ; le sébacate de diisopropyle ; l'adipate de diisopropyle ; le citrate de triisopropyle ;
le néopentanoate d'isostéaryle, le néopentanoate de tridécyle.

Parmi les esters cités ci-dessus, on préfère utiliser le palmitate d'isopropyle, le palmitate d'éthyl-2-héxyle, les myristates d'isopropyle, de t-butyle, l'isostéarate de butyle, le stéarate d'isobutyle ;l'isononanate d'isononyle et le néopentanoate d'isostéaryle.

Les esters selon l'invention sont de préférence des monoesters d'acide carboxylique et de monoalcool.

Le ou les esters d'acide carboxylique peuvent être utilisées dans les compositions conformes à l'invention dans des concentrations allant généralement de 0,5 à 5 % en poids par rapport au poids total de la composition et encore plus particulièrement de 0,8 à 3% en poids.

Selon un mode de réalisation de l'invention, les compositions peuvent comprendre en outre un sel hydrosoluble et/ou un alcool hydrosoluble mono ou polyhydroxylé Les sels hydrosolubles selon l'invention sont de préférence les sels de métaux mono ou divalents et d'un acide minéral ou organique.
On peut citer en particulier le chlorure de sodium, le chlorure de potassium, le chlorure de calcium, le sulfate de magnésium, le citrate de sodium, les sels de sodium de l'acide phosphorique. Le chlorure de sodium est particulièrement préféré.

Les compositions détergentes selon l'invention présentent un pH final généralement compris entre 3 et 8. De préférence, ce pH est compris entre 4 et 7,5. L'ajustement du pH à la valeur désirée peut se faire classiquement par ajout d'une base (organique ou minérale) dans la composition, par exemple de la soude, de l'ammoniaque ou une (poly)amine primaire, secondaire ou tertiaire comme la monoéthanolamine, la diéthanolamine, la triéthanolamine, l'isopropanolamine ou la propanediamine-1,3, ou encore par ajout d'un acide minéral ou organique, de préférence l'acide citrique ou l'acide chlorhydrique.

Le milieu aqueux cosmétiquement acceptable peut être constitué uniquement par de l'eau ou par un mélange d'eau et d'un solvant cosmétiquement acceptable, tel qu'un alcool inférieur en C₁-C₄, comme l'éthanol, l'isopropanol, le tertiobutanol, le n-butanol ; les alkylèneglycols comme le propylèneglycol, l'hexylèneglycol ou le glycérol.

La composition selon l'invention comprend de préférence au moins 30% en poids d'eau et plus particulièrement de 50 à 90% en poids et encore préférentiellement de 70 à 85% en poids par rapport au poids total de la composition.

La composition comprend de préférence moins de 20% en poids de phase grasse.

La phase grasse comprend tous les corps gras insoluble dans l'eau à température ambiante de la composition tels que notamment les esters gras, les huiles végétales, minérales, synthétiques, les alcools gras, les acides gras, les amides gras, les cires, les silicones. La phase grasse représente de préférence de 0,1 à 15% en poids et plus particulièrement de 0,5 à 10% en poids par rapport au poids total de la composition et encore plus particulièrement de 0,5 à 8% en poids.

Les compositions conformes à l'invention peuvent contenir en plus de l'association définie ci-dessus des agents régulateurs de viscosité tels que des agents épaississants. On peut citer en particulier les scléroglucanes, les gommes de xanthane, les alcanolamides d'acide gras, les alcanolamides d'acide alkyl éther carboxylique éventuellement oxyéthylénés avec jusqu'à 5 moles d'oxyde d'éthylène tel que le produit commercialisé sous la dénomination "AMINOL A15" par la société CHEM Y, les acides polyacryliques réticulés et les copolymères acide acrylique / acrylates d'alkyle en C₁₀-C₃₀ réticulés. Ces agents régulateurs de viscosité sont utilisés dans les compositions selon l'invention dans des proportions pouvant aller jusqu'à 10 % en poids par rapport au poids total de la composition.

Les compositions conformes à l'invention peuvent également contenir jusqu'à 5 % d'agents nacrants ou opacifiants bien connus dans l'état de la technique tels que par exemple les alcools gras, les palmitates de sodium ou de magnésium, les stéarates et hydroxystéarates de sodium ou de magnésium, les alcool gras, les dérivés acylés à chaîne grasse tels que les distéarates d'éthylène glycol ou de polyéthylèneglycol, les éthers à chaînes grasses tels que par exemple le distéaryléther ou le 1-(hexadécyloxy)-2-octadécanol.

Les compositions conformes à l'invention peuvent éventuellement contenir en outre d'autres agents ayant pour effet d'améliorer les propriétés cosmétiques de cheveux ou de la peau sans cependant altérer la stabilité des compositions. On peut citer à ce sujet les agents tensioactifs cationiques, les polymères anioniques ou non ioniques ou cationiques ou amphotères, les protéines, les hydrolysats de protéines, les céramides, les pseudocéramides, les huiles végétales, les acides gras notamment à chaînes linéaires ou ramifiées en C₁₆-C₄₀ tels que l'acide méthyl-18 eicosanoïque, les hydroxyacides, les vitamines, les provitamines telle que le panthénol, les silicones, volatiles ou non volatiles, solubles et insolubles dans le milieu, les filtres UV, les agents hydratants, les agents antipelliculaires ou antiséborrhéiques, les agents antichute des cheveux, les agents anti-radicaux libres, et leurs mélanges.

Selon un mode particulièrement préféré, les compositions selon l'invention comprennent en outre au moins un polymère cationique.

Les polymères cationiques utilisables conformément à la présente invention peuvent être choisis parmi tous ceux déjà connus en soi comme améliorant les propriétés cosmétiques des cheveux traités par des compositions détergentes, à savoir notamment ceux décrits dans la demande de brevet EP-A-0 337 354 et dans les demandes de brevets français FR-A-2 270 846, 2 383 660, 2 598 611, 2 470 596 et 2 519 863.

De manière encore plus générale, au sens de la présente invention, l'expression « polymère cationique » désigne tout polymère contenant des groupements cationiques et/ou des groupements ionisables en groupements cationiques.

Les polymères cationiques utilisables selon l'invention ont de préférence une densité de charge cationique supérieure ou égale à 0,2 meq./g, et plus particulièrement comprise entre 0,2 et 8,5 meq./g.

Parmi tous les polymères cationiques susceptibles d'être utilisés dans le cadre de la présente invention, on préfère mettre en oeuvre les dérivés d'éther de cellulose quaternaires tels que les produits commercialisés sous la dénomination « JR 400 » par la société UNION CARBIDE CORPORATION, les cyclopolymères, en particulier les homopolymères de sel de diallyldiméthylammonium et les copolymères de sel de diallyldiméthylammonium et d'acrylamide en particulier les chlorures, commercialisés sous les dénominations «MERQUAT 100 », « MERQUAT 550 » et « MERQUAT S » par la société MERCK, les polysaccharides cationiques et plus particulièrement les gommes de guar modifiées par du chlorure de 2,3-époxypropyl triméthylammonium commercialisées par exemple sous la dénomination « JAGUAR C13S » par la société MEYHALL, les homopolymères et les copolymères éventuellement réticulés de sel de (méth)acryloyloxyéthyltriméthylammonium, vendus par la société ALLIED COLLOIDS en solution à 50% dans de l'huile minérale sous les dénominations commerciales SALCARE SC92 (copolymère réticulé du chlorure de méthacryloyloxyéthyltriméthylammonium et de l'acrylamide) et SALCARE SC95 (homopolymère réticulé du chlorure de méthacryloyloxyéthyl triméthylammonium), les copolymères quaternaires de vinylpyrrolidone et de sel de vinyl imidazole tels que les produits commercialisés par BASF sous les dénominations LUVIQUAT FC 370, LUVIQUAT FC 550, LUVIQUAT FC 905 et LUVIQUAT HM-552.

On peut également utiliser les polymères qui sont constitués de motifs récurrents répondant à la formule : 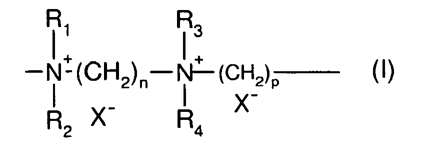 dans laquelle R₁, R₂, R₃ et R₄, identiques ou différents, désignent un radical alkyle ou hydroxyalkyle ayant de 1 à 4 atomes de carbone environ, n et p sont des nombres entiers variant de 2 à 20 environ et, X⁻ est un anion dérivé d'un acide minéral ou organique.

Un composé de formule (I) particulièrement préféré est celui pour lequel R₁, R₂, R₃ et R₄, représentent un radical méthyle et n = 3, p = 6 et X = Cl, dénommé Hexadimethrine chloride selon la nomenclature INCl (CTFA).

Selon l'invention, le ou les polymères cationiques peuvent représenter de 0,001 % à 10 % en poids, de préférence de 0,005 % à 5% en poids, et encore plus préférentiellement de 0,01 % à 3 % en poids, du poids total de la composition finale.

Les compositions selon l'invention peuvent contenir également des synergistes de mousses tels que des 1,2-alcanediols en C₁₀-C₁₈ ou des alcanolamides gras en C₁₀-C₁₈ dérivés de mono ou de diéthanolamine.

Bien entendu, l'homme du métier veillera à choisir ce ou ces éventuels composés complémentaires et/ou leurs quantités de manière telle que la solubilité des esters d'acide carboxylique selon l'invention, la stabilité des composition et les propriétés cosmétiques attachées intrinsèquement à la composition conforme à l'invention ne soient pas, ou substantiellement pas, altérées par la ou les adjonctions envisagées. L'addition de certains composés tels que les agents nacrants peut rendre la composition non transparente.

La transparence peut se mesurer par mesure de la transmittance à 700nm via un spectromètre (par exemple spectromètre Lambda 14 de Perkin Elmer ou UV21 01 PC de Shimadzu) . Les compositions transparentes ont une transmittance supérieure ou égale à 94%, de préférence de 96 à 100%.

Le pouvoir moussant des compositions selon l'invention, caractérisé par une hauteur de mousse, est généralement supérieur à 75 mm ; de préférence, supérieure à 100 mm mesurée selon la méthode ROSS-MILES (NF T 73-404 /ISO696) modifiée.

### Les modifications de la méthode sont les suivantes :

La mesure se fait à la température de 22°C avec de l'eau osmosée. La concentration de la solution est de 2g/l. La hauteur de la chute est de 1 m. La quantité de composition qui chute est de 200 ml. Ces 200 ml de composition tombe dans une éprouvette ayant un diamètre de 50 mm et contenant 50 ml de la composition à tester. La mesure est faite 5 minutes après l'arrêt de l'écoulement de la composition.

Ces compositions peuvent se présenter sous la forme de liquides plus ou moins épaissis, de crèmes ou de gels et elles conviennent principalement au lavage, au soin des matières kératiniques en particulier des cheveux et de la peau et encore plus particulièrement des cheveux.

L'invention a également pour objet un procédé de lavage et de conditionnement des matières kératiniques telles que notamment les cheveux consistant à appliquer sur lesdites matières mouillées une quantité efficace d'une composition telle que définie ci-dessus, puis à effectuer un rinçage à l'eau après un éventuel temps de pause.

Les compositions selon l'invention sont utilisées de préférence comme shampooings pour le lavage et le conditionnement des cheveux et ils sont appliqués dans ce cas-là sur les cheveux humides dans des quantités efficaces pour les laver, et la mousse générée par massage ou friction avec les mains est ensuite éliminée après un éventuel temps de pause, par rinçage avec de l'eau, l'opération pouvant être répétée une ou plusieurs fois.

Les compositions conformes à l'invention sont également utilisables comme gels douche pour le lavage et le conditionnement des cheveux et/ou de la peau, auquel cas ils sont appliqués sur la peau et/ou les cheveux humides et sont rincés après application.

Des exemples concrets, mais nullement limitatifs, illustrant l'invention vont maintenant être donnés.

### EXEMPLES 1 et 2

On a réalisé les compositions de shampooing conformes à l'invention suivantes :

| | 1 | 2 |
|---|---|---|
| - Lauryléthersulfate de sodium (C12/C14 à 70/30) à 2,2 moles d'oxyde d'éthylène en solution aqueuse à 70% de MA | 14,2 gMA | 14,2 gMA |
| - Cocoylbétaïne à 30% MA (DEHYTON AB 30 de COGNIS) | 1,9 gMA | 1,9 gMA |
| Acide lauryléther carboxylique (AKYPO RLM 45 CA de KAO) | 1,8 gMA | 1,8 gMA |
| - Myristate d'isopropyle | 2 g | - |
| - Néopentanoate d'isostéaryle | - | 1 g |
| - Cellulose cationique (JR400 de AMERCHOL) | 0,3 g | 0,3 g |
| - Monoisopropanolamide d'acide de coprah | 3,3 g | 3,3 g |
| - Parfum, conservateur | qs | qs |
| - Acide chlorhydrique qs pH | 5-5,6 | 5-5,6 |
| - Eau déminéralisée qsp | 100 g | 100 g |
| | | |
| Transmittance | >94% | >94% |

Les compositions selon l'invention sont transparentes et stables.
Lorsque les compositions sont appliquées sur des cheveux humides, la mousse se développe rapidement, elle est abondante et se rince facilement.
Les cheveux traités avec ces compositions se démêlent facilement et sont lisses de la racine à la pointe. Ils ont un toucher propre et sont « corporisés ».

### EXEMPLE 3

On a réalisé la composition de shampooings, conforme à l'invention

| | |
|---|---|
| - Lauryléthersulfate de sodium (C12/C14 à 70/30) à 2,2 moles d'oxyde d'éthylène en solution aqueuse à 70% de MA | 10 gMA |
| Sodium méthyl cocoyl taurate à 30% dans l'eau (HOSTAPON LT-SF de CLARIANT) | 3 gMA |
| Cocoylglutamate de triéthanolamine à 30% dans l'eau (AMISOFT CT 12 d'AJINOMOTO) | 2 gMA |
| - Disodium cocodiamphodiacetate à39% de matière active (MIRANOL C2M CONC de RHODIA) | 3 gMA |
| Acide lauryléther carboxylique (AKYPO RLM 45 CA de KAO) | 1,8 gMA |
| - Myristate d'isopropyle | 2 g |
| - Cellulose cationique (JR400 de AMERCHOL) | 0,3 g |
| - Monoisopropanolamide d'acide de coprah | 3,3 g |
| - Parfum, conservateur | qs |
| - Acide chlorhydrique qs pH | 5-5,6 |
| - Eau déminéralisée qsp | 100 g |
| | |
| Transmittance | >94% |

On a obtenu des résultats similaires à ceux des exemples 1 et 2.

### EXEMPLES 4 et 5

On réalise les compositions de shampooing conformes à l'invention suivantes :

| | 4 | 5 |
|---|---|---|
| - Lauryléthersulfate de sodium (C12/C14 à 70/30) à 2,2 moles d'oxyde d'éthylène en solution aqueuse à 70% de MA | 12,6 gMA | 12,6 gMA |
| - Cocoylbétaïne à 30% MA (DEHYTON AB 30 de COGNIS) | 2,07 gMA | 2,07 gMA |
| Acide lauryléther carboxylique à 10 moles d'oxyde diéthylène (AKYPO RLM 100 de KAO) | 1,8 gMA | 3,6 gMA |
| - Néopentanoate d'isostéaryle | 1 g | 1 g |
| - Cellulose cationique (JR400 de AMERCHOL) | 0,27 g | 0,27 g |
| - Monoisopropanolamide d'acide de coprah | 2,94 g | 2,94 g |
| - Parfum, conservateur | qs | qs |
| - Acide chlorhydrique qs pH | 5-5,6 | 5-5,6 |
| - Eau déminéralisée qsp | 100 g | 100 g |
| | | |
| Transmittance | >94% | >94% |

On obtient des résultats similaires à ceux des exemples 1 et 2.

### EXEMPLE 6

On réalise les compositions de shampooing conformes à l'invention suivantes :

| | 6 |
|---|---|
| - Laurylsulfate d'ammonium (C12/C14 à 70/30) en solution aqueuse à 70% de MA | 12,6 gMA |
| - Cocoylbétaïne à 30% MA (DEHYTON AB 30) | 2,07 gMA |
| Acide lauryléther carboxylique (AKYPO RLM 45 CA de KAO) | 1,8 gMA |
| - Néopentanoate d'isostéaryle | 1 g |
| - Cellulose cationique (JR400 de AMERCHOL) | 0,27 g |
| - Monoisopropanolamide d'acide de coprah | 2,94 g |
| - Parfum, conservateur | qs |
| - Acide chlorhydrique qs pH | 5-5,6 |
| - Eau déminéralisée qsp | 100 g |
| | |
| Transmittance | >94% |

On obtient des résultats similaires à ceux des exemples 1 et 2.

### EXEMPLE 7

On réalise les compositions de shampooing conformes à l'invention suivantes :

| | 7 |
|---|---|
| - Lauryléthersulfate de sodium (C12/C14 à 70/30) à 2,2 moles d'oxyde d'éthylène en solution aqueuse à 70% de MA | 12,6 gMA |
| - Cocoylbétaïne à 30% MA (DEHYTON AB 30 de COGNIS) | 4,14 gMA |
| Acide lauryléther carboxylique (AKYPO RLM 45 CA de KAO) | 1,8 gMA |
| - Myristate d'isopropyle | 1 g |
| - Cellulose cationique (JR400 de AMERCHOL) | 0,27 g |
| - Monoisopropanolamide d'acide de coprah | 2,94 g |
| - Parfum, conservateur | qs |
| - Acide chlorhydrique qs pH | 5-5,6 |
| - Eau déminéralisée qsp | 100 g |
| | |
| Transmittance | >94% |

On obtient des résultats similaires à ceux des exemples 1 et 2.

## Revendications

1. Composition cosmétique détergente, **caractérisée par le fait qu'**elle comprend, dans un milieu aqueux cosmétiquement acceptable, (A) au moins un tensioactif anionique sulfate ou sulfonate, (B) au moins un tensioactif anionique carboxylique différent du tensioactif cité en (A) choisi parmi les acides alkyl (C₆-C₂₄) éther carboxyliques polyoxyalkylénés, les acides alkyl(C₆-C₂₄)aryl éther carboxyliques polyoxyalkylénés et leurs sels, les acides alkyl(C₆-C₂₄) amido éther carboxyliques polyoxyalkylénés et leurs sels, (C) au moins un tensioactif amphotère, et (D) au moins un ester d'acide carboxylique insoluble dans l'eau lesdits esters sont choisis parmi :
1) les esters d'acide carboxylique en C3-C30 et d'alcool en C1-C30, l'un au moins de l'acide ou de l'alcool étant ramifié, et
2) les esters d'acide aromatique en C7-C30 dont la fonction carboxylique est directement liée au cycle aromatique et d'alcool en C1-C30,
la quantité d'esters allant de 0,5 à 10% en poids par rapport au poids total de la composition,
le rapport en poids tensioactif anionique sulfate ou sulfonate / tensioactif anionique carboxylique va de 2 à 12.

2. Composition selon la revendication 1, **caractérisée par le fait que** la quantité totale de tensioactif va de 4 % à 50 % en poids par rapport au poids total de la composition, de préférence de 6 % à 35 % en poids et plus préférentiellement de 8 % à 25 % en poids et encore plus particulièrement de 14 à 20 % en poids.

3. Composition selon l'une quelconque des revendications 1 ou 2, **caractérisée en ce que** le(s) tensioactif(s) anionique(s) sulfate ou sulfonate est (sont) présent(s) dans des concentrations allant de 1,5 à 50% en poids, de préférence de 2 à 25 % en poids, et plus particulièrement de 8 à 20% en poids et encore plus préférentiellement de 10 à 16% en poids par rapport au poids total de la composition.

4. Composition selon l'une quelconque des revendications 1 à 3, **caractérisée en ce que** le(s) tensioactif(s) amphotère(s) est(sont) présent(s) dans des concentrations allant de 0,1 à 20 % en poids, de préférence de 1 à 15 % en poids, plus particulièrement de 1,5 à 5% en poids par rapport au poids total de la composition.

5. Composition selon l'une quelconque des revendications 1 à 4, **caractérisée en ce que** ledit(s) tensioactif(s) anionique(s) carboxylique(s) est(sont) présent(s) dans des concentrations allant de 0,5 à 15 % en poids, de préférence de 1 à 10 % en poids, et encore plus particulièrement de 1,5 à 5% en poids par rapport au poids total de la composition.

6. Composition selon l'une quelconque des revendications 1 à 5, **caractérisée par le fait que** le rapport en poids tensioactif anionique sulfate ou sulfonate / tensioactif amphotère va de 2 à 12, plus particulièrement de 4 à 10.

7. Composition selon l'une quelconque des revendications 1 à 6, **caractérisée par le fait que** le rapport en poids tensioactif anionique sulfate ou sulfonate / tensioactif anionique carboxylique va de 4 à 10.

8. Composition selon l'une quelconque des revendications 1 à 7, **caractérisée par le fait que** le rapport en poids tensioactif anionique carboxylique/ tensioactif amphotère va de 0,3 à 3 et encore plus préférentiellement de 0,5 à 1,5.

9. Composition selon l'une quelconque des revendications 1 à 8, **caractérisée par le fait que** lesdits esters sont choisis parmi :
les esters d'acide carboxylique ramifié ayant de 4 à 6 atomes de carbone et d'alcool ayant de 8 à 26 atomes de carbone,
les esters d'acide carboxylique linéaire ayant de 12 à 26 atomes de carbone et d'alcool ramifié ayant de 3 à 12 atomes de carbone,
les esters d'acide carboxylique linéaire ayant de 2 à 12 atomes de carbone et d'alcool ramifié ayant de 8 à 26 atomes de carbone.
les esters d'acide carboxylique ramifié ayant de 8 à 26 atomes de carbone, de préférence 8 à 12 et d'alcool ramifié ayant de 8 à 26 atomes de carbone, de préférence 8 à 12,

10. Composition selon l'une quelconque des revendications 1 à 9, **caractérisée par le fait que** lesdits esters sont choisis parmi :
le lactate d'isostéaryle ; l'octanoate d'isostéaryle ; l'octanoate d'isocétyle ; l'octanoate d'isodécyle ; l'isononanoate d'isononyle ; l'isononanoate d'octyle ; l'isononate de 2-éthylhexyle ; le palmitate d'isopropyle, le palmitate d'éthyl-2-hexyle, le myristate d'isopropyle, le myristate de tertio-butyle, le stéarate d'isobutyle ; le sébacate de diisopropyle ; l'adipate de diisopropyle ; le citrate de triisopropyle ; le néopentanoate d'isostéaryle, le néopentanoate de tridécyle.

11. Composition selon la revendication 10, **caractérisée par le fait que** l'ester est choisi parmi le palmitate d'isopropyle, le palmitate d'éthyl-2-héxyle, les myristates d'isopropyle, de tertio-butyle, l'isostéarate de butyle, le stéarate d'isobutyle, l'isononanate d'isononyle et le néopentanoate d'isostéaryle.

12. Composition selon la revendication 11, **caractérisée par le fait que** l'ester est choisi parmi le palmitate d'isopropyle, les myristates d'isopropyle, de tertio-butyle, l'isononanate d'isononyle et le néopentanoate d'isostéaryle.

13. Composition selon l'une quelconque des revendications 1 à 12, **caractérisée par le fait que** lesdits esters sont présents dans des concentrations allant de 0,5 à 5 % en poids par rapport au poids total de la composition et encore plus particulièrement de 0,8 à 3% en poids.

14. Composition selon l'une quelconque des revendications 1 à 13, **caractérisée par le fait que** la composition comprend en outre au moins un polymère cationique.

15. Composition selon la revendication 14, **caractérisée par le fait que** le polymère cationique est choisi parmi les dérivés d'éther de cellulose quaternaires, les 29 homopolymères de sel de diallyldiméthylammonium et les copolymères de sel de diallyldiméthylammonium et d'acrylamide, les polysaccharides cationiques, les copolymères quaternaires de vinylpyrrolidone et de sel de vinyl imidazole.

16. Composition selon la revendication 15, **caractérisée par le fait que** le polymère cationique est choisi parmi les polymères qui sont constitués de motifs récurrents répondant à la formule : 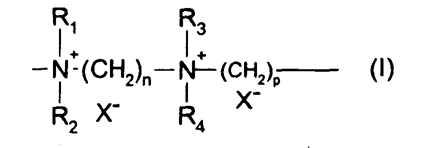 dans laquelle R₁, R₂, R₃ et R₄, identiques ou différents, désignent un radical alkyle ou hydroxyalkyle ayant de 1 à 4 atomes de carbone environ, n et p sont des nombres entiers variant de 2 à 20 environ et, X- est un anion dérivé d'un acide minéral ou organique.

17. Composition selon l'une quelconque des revendications 15 à 16, **caractérisée en ce que** ledit polymère cationique représente de 0,001 % à 10 % en poids, de préférence de 0,005 % à 5 % en poids, et encore plus préférentiellement de 0,01% à 3 % en poids, du poids total de la composition.

18. Composition selon l'une quelconque des revendications 1 à 16, **caractérisée par le fait que** le milieu aqueux cosmétiquement acceptable est constitué uniquement par de l'eau ou par un mélange d'eau et d'un solvant cosmétiquement acceptable.

19. Composition selon la revendication 18, **caractérisée par le fait que** le solvant est choisi parmi les alcools inférieurs en C₁-C₄, comme l'éthanol, l'isopropanol, le tertiobutanol, le n-butanol ; les alkylèneglycols comme le propylèneglycol ou l'hexylèneglycol, le glycérol.

20. Composition selon l'une quelconque des revendications 1 à 19, **caractérisée par le fait qu'**elle contient en plus un ou plusieurs adjuvants choisis parmi les agents tensioactifs cationiques, les polymères anioniques ou non ioniques ou amphotères, les protéines, les hydrolysats de protéines, les céramides, les pseudocéramides, les huiles végétales, les acides gras, les hydroxyacides, les vitamines, les provitamines telle que le panthénol, les silicones volatiles ou non volatiles, solubles ou insolubles dans le milieu, les filtres UV, les agents hydratants, les agents antipelliculaires ou antiséborrhéiques, les agents antichute des cheveux, les agents anti-radicaux libres, les agents opacifiants et leurs mélanges.

21. Utilisation d'une composition telle que définie dans l'une quelconque des revendications 1 à 20 pour le nettoyage et/ou le démaquillage des matières kératiniques.

22. Procédé de lavage et de conditionnement des matières kératiniques telles que les cheveux consistant à appliquer sur lesdites matières mouillées une quantité efficace d'une composition telle que définie dans l'une quelconque des revendications 1 à 20, puis à effectuer un rinçage à l'eau après un éventuel temps de pause.

## Claims

1. Detergent cosmetic composition, **characterized in that** it comprises, in a cosmetically acceptable aqueous medium, (A) at least one sulfate or sulfonate anionic surfactant, (B) at least one carboxylic anionic surfactant other than the surfactant mentioned in (A), chosen from polyoxyalkylenated (C₆-C₂₄)alkyl ether carboxylic acids, polyoxyalkylenated (C₆-C₂₄) alkylaryl ether carboxylic acids and salts thereof, polyoxyalkylenated (C₆-C₂₄)alkylamido ether carboxylic acids and salts thereof, (C) at least one amphoteric surfactant and (D) at least one water-insoluble carboxylic acid ester, the said esters are chosen from:
1) esters of a C3-C30 carboxylic acid and of a C1-C30 alcohol, at least the acid or the alcohol being branched, and
2) esters of a C7-C30 aromatic acid whose carboxylic function is directly attached to the aromatic ring and of a C1-C30 alcohol,
the amount of esters ranging from 0.5% to 10% by weight relative to the total weight of the composition,
the sulfate or sulfonate anionic surfactant/carboxylic anionic surfactant weight ratio ranges from 2 to 12.

2. Composition according to Claim 1, **characterized in that** the total amount of surfactant ranges from 4% to 50% by weight, preferably from 6% to 35% by weight, more preferentially from 8% to 25% by weight and even more particularly from 14% to 20% by weight relative to the total weight of the composition.

3. Composition according to either of Claims 1 and 2, **characterized in that** the sulfate or sulfonate anionic surfactant(s) is (are) present in concentrations ranging from 1.5% to 50% by weight, preferably from 2% to 25% by weight, more particularly from 8% to 20% by weight and even more preferentially from 10% to 16% by weight relative to the total weight of the composition.

4. Composition according to any one of Claims 1 to 3, **characterized in that** the amphoteric surfactant(s) is (are) present in concentrations ranging from 0.1% to 20% by weight, preferably from 1% to 15% by weight and more particularly from 1.5% to 5% by weight relative to the total weight of the composition.

5. Composition according to any one of Claims 1 to 4, **characterized in that** the said carboxylic anionic surfactant(s) is (are) present in concentrations ranging from 0.5% to 15% by weight, preferably from 1% to 10% by weight and even more particularly from 1.5% to 5% by weight relative to the total weight of the composition.

6. Composition according to any one of Claims 1 to 5, **characterized in that** the sulfate or sulfonate anionic surfactant/amphoteric surfactant weight ratio ranges from 2 to 12 and more particularly from 4 to 10.

7. Composition according to any one of Claims 1 to 6, **characterized in that** the sulfate or sulfonate anionic surfactant/carboxylic anionic surfactant weight ratio ranges from 4 to 10.

8. Composition according to any one of Claims 1 to 7, **characterized in that** the carboxylic anionic surfactant/amphoteric surfactant weight ratio ranges from 0.3 to 3 and even more preferentially from 0.5 to 1.5.

9. Composition according to any one of Claims 1 to 8, **characterized in that** the said esters are chosen from:
esters of a branched carboxylic acid containing from 4 to 6 carbon atoms and of an alcohol containing from 8 to 26 carbon atoms,
esters of a linear carboxylic acid containing from 12 to 26 carbon atoms and of a branched alcohol containing from 3 to 12 carbon atoms,
esters of a linear carboxylic acid containing from 2 to 12 carbon atoms and of a branched alcohol containing from 8 to 26 carbon atoms,
esters of a branched carboxylic acid containing from 8 to 26 and preferably from 8 to 12 carbon atoms and of a branched alcohol containing from 8 to 26 and preferably 8 to 12 carbon atoms.

10. Composition according to any one of Claims 1 to 9, **characterized in that** the said esters are chosen from: isostearyl lactate; isostearyl octanoate; isocetyl octanoate; isodecyl octanoate; isononyl isononanoate; octyl isononanoate; 2-ethylhexl isononate; isopropyl palmitate, 2-ethylhexyl palmitate, isopropyl myristate, tert-butyl myristate, isobutyl stearate; diisopropyl sebacate; diisopropyl adipate; triisopropyl citrate; isostearyl neopentanoate, tridecyl neopentanoate.

11. Composition according to Claim 10, **characterized in that** the ester is chosen from isopropyl palmitate, 2-ethylhexyl palmitate, isopropyl or tert-butyl myristate, butyl isostearate, isobutyl stearate, isononyl isononanoate and isostearyl neopentanoate.

12. Composition according to Claim 11, **characterized in that** the ester is chosen from isopropyl palmitate, isopropyl myristate, tert-butyl myristate, isononyl isononanoate and isostearyl neopentanoate.

13. Composition according to any one of Claims 1 to 12, **characterized in that** the said esters are present in concentrations ranging from 0.5% to 5% by weight and even more particularly from 0.8% to 3% by weight relative to the total weight of the composition.

14. Composition according to any one of Claims 1 to 13, **characterized in that** the composition also comprises at least one cationic polymer.

15. Composition according to Claim 14, **characterized in that** the cationic polymer is chosen from quaternary cellulose ether derivatives, diallyldimethylammonium salt homopolymers and copolymers of a diallyldimethylammonium salt and of acrylamide, cationic polysaccharides and quaternary copolymers of vinylpyrrolidone and of a vinylimidazole salt.

16. Composition according to Claim 15, **characterized in that** the cationic polymer is chosen from polymers that consist of repeating units corresponding to the formula: 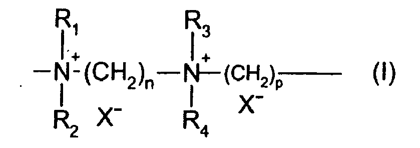 in which R₁, R₂, R₃ and R₄, which may be identical or different, denote an alkyl or hydroxyalkyl radical containing from 1 to 4 carbon atoms approximately, n and p are integers ranging from 2 to 20 approximately, and X⁻ is an anion derived from a mineral or organic acid.

17. Composition according to either of Claims 15 and 16, **characterized in that** the said cationic polymer represents from 0.001% to 10% by weight, preferably from 0.005% to 5% by weight and even more preferentially from 0.0.1% to 3% by weight relative to the total weight of the composition.

18. Composition according to any one of Claims 1 to 16, **characterized in that** the cosmetically acceptable aqueous medium consists solely of water or of a mixture of water and of a cosmetically acceptable solvent.

19. Composition according to Claim 18, **characterized in that** the solvent is chosen from C₁-C₄ lower alcohols, for instance ethanol, isopropanol, tert-butanol or n-butanol; alkylene glycols, for instance propylene glycol or hexylene glycol, and glycerol.

20. Composition according to any one of Claims 1 to 19, **characterized in that** it also contains one or more adjuvants chosen from cationic surfactants, anionic, nonionic or amphoteric polymers, proteins, protein hydrolysates, ceramides, pseudoceramides, plant oils, fatty acids, hydroxy acids, vitamins, provitamins such as panthenol, volatile or non-volatile silicones, which are soluble or insoluble in the medium, UV-screening agents, moisturizers, antidandruff or anti-seborrhoeic agents, hair-loss counteractants, free-radical scavengers and opacifiers, and mixtures thereof.

21. Use of a composition as defined in any one of Claims 1 to 20, for cleansing and/or removing makeup from keratin materials.

22. Process for washing and conditioning keratin materials such as the hair, which consists in applying to the said wet materials an effective amount of a composition as defined- in any one of Claims 1 to 20, and then in rinsing with water after an optional leave-in time.

## Patentansprüche

1. Reinigende kosmetische Zusammensetzung, **dadurch gekennzeichnet, dass** sie in einem kosmetisch akzeptablen wässrigen Medium (A) mindestens ein anionisches grenzflächenaktives Sulfat oder Sulfonat, (B) mindestens eine anionische grenzflächenaktive Carbonsäure, die von dem unter (A) genannten grenzflächenaktiven Stoff verschieden und unter den polyalkoxylierten Alkyl(C₆₋₂₄)ethercarbonsäuren, polyalkoxylierten Alkyl(C₆₋₂₄)arylethercarbonsäuren und ihren Salzen, polyalkoxylierten Alkyl(C₆₋₂₄)amidoethercarbonsäuren und deren Salzen ausgewählt ist, (C) mindestens einen amphoteren grenzflächenaktiven Stoff und (D) mindestens einen in Wasser unlöslichen Carbonsäureester enthält, wobei die Ester ausgewählt sind unter:
1) Estern einer C₃₋₃₀-Carbonsäure und eines C₁₋₃₀-Alkohols, wobei zumindest die Säure oder der Alkohol verzweigt vorliegt,
2) Estern einer aromatischen C₇₋₃₀-Carbonsäure, bei der die Carboxyfunktion direkt an den aromatischen Ring gebunden ist, und eines C₁₋₃₀-Alkohols,
wobei der Mengenanteil des Esters im Bereich von 0,5 bis 10 Gew.-%, bezogen auf das Gesamtgewicht der Zusammensetzung, liegt, und
das Gewichtsverhältnis des anionischen grenzflächenaktiven Sulfats oder Sulfonats und der anionischen grenzflächenaktiven Carbonsäure im Bereich von 2 bis 12 liegt.

2. Zusammensetzung nach Anspruch 1, **dadurch gekennzeichnet, dass** die Gesamtmenge an grenzflächenaktivem Stoff im Bereich von 4 bis 50 Gew.-%, bezogen auf das Gesamtgewicht der Zusammensetzung, vorzugsweise im Bereich von 6 bis 35 Gew.-%, insbesondere im Bereich von 8 bis 25 Gew.-% und besonders bevorzugt im Bereich von 14 bis 20 Gew.-% liegt.

3. Zusammensetzung nach einem der Ansprüche 1 oder 2, **dadurch gekennzeichnet, dass** das oder die anionische(n) grenzflächenaktive(n) Sulfat(e) oder Sulfonat(e) in Konzentrationen von 1,5 bis 50 Gew.-%, vorzugsweise 2 bis 25 Gew.-%, insbesondere 8 bis 20 Gew.-% und besonders bevorzugt 10 bis 16 Gew.-%, bezogen auf das Gesamtgewicht der Zusammensetzung, enthalten ist (sind).

4. Zusammensetzung nach einem der Ansprüche 1 bis 3, **dadurch gekennzeichnet, dass** der oder die amphotere(n) grenzflächenaktive(n) Stoff(e) in Konzentrationen von 0,1 bis 20 Gew.-%, vorzugsweise 1 bis 15 Gew.-% und insbesondere 1,5 bis 5 Gew.-%, bezogen auf das Gesamtgewicht der Zusammensetzung, enthalten ist (sind).

5. Zusammensetzung nach einem der Ansprüche 1 bis 4, **dadurch gekennzeichnet, dass** die anionische(n) grenzflächenaktive(n) Carbonsäure(n) in Konzentrationen von 0,5 bis 15 Gew.-%, vorzugsweise 1 bis 10 Gew.-% und insbesondere 1,5 bis 5 Gew.-%, bezogen auf das Gesamtgewicht der Zusammensetzung, enthalten ist (sind).

6. Zusammensetzung nach einem der Ansprüche 1 bis 5, **dadurch gekennzeichnet, dass** das auf das Gewicht bezogene Verhältnis des anionischen grenzflächenaktiven Sulfats oder Sulfonats und des amphoteren grenzflächenaktiven Stoffes im Bereich von 2 bis 12 und insbesondere im Bereich von 4 bis 10 liegt.

7. Zusammensetzung nach einem der Ansprüche 1 bis 6, **dadurch gekennzeichnet, dass** das auf das Gewicht bezogene Verhältnis anionisches grenzflächenaktives Sulfat oder Sulfonat/anionische grenzflächenaktive Carbonsäure im Bereich von 4 bis 10 liegt.

8. Zusammensetzung nach einem der Ansprüche 1 bis 7, **dadurch gekennzeichnet, dass** das auf das Gewicht bezogene Verhältnis anionische grenzflächenaktive Carbonsäure/amphoterer grenzflächenaktiver Stoff im Bereich von 0,3 bis 3 und noch bevorzugter im Bereich von 0,5 bis 1,5 liegt.

9. Zusammensetzung nach einem der Ansprüche 1 bis 8, **dadurch gekennzeichnet, dass** die Ester ausgewählt sind unter:
Estern einer verzweigten Carbonsäure mit 4 bis 6 Kohlenstoffatomen und eines Alkohols mit 8 bis 26 Kohlenstoffatomen,
Estern einer linearen Carbonsäure mit 12 bis 26 Kohlenstoffatomen und eines verzweigten Alkohols mit 3 bis 12 Kohlenstoffatomen,
Estern einer linearen Carbonsäure mit 2 bis 12 Kohlenstoffatomen und eines verzweigten Alkohols mit 8 bis 26 Kohlenstoffatomen,
Estern einer verzweigten Carbonsäure mit 8 bis 26 Kohlenstoffatomen und vorzugsweise 8 bis 12 Kohlenstoffatomen und eines verzweigten Alkohols mit 8 bis 26 Kohlenstoffatomen und vorzugsweise 8 bis 12 Kohlenstoffatomen.

10. Zusammensetzung nach einem der Ansprüche 1 bis 9, **dadurch gekennzeichnet, dass** die Ester ausgewählt sind unter:
Isostearyllactat; Isostearyloctanoat; Isocetyloctanoat;
Isodecyloctanoat; Isononylisononanoat; Octylisononanoat; 2-Ethylhexylisononat; Isopropylpalmitat; 2-Ethylhexylpalmitat;
Isopropylmyristat; tert-Butylmyristat; Isobutylstearat;
Diisopropylsebacat; Diisopropyladipat; Triisopropylcitrat;
Isostearylneopentanoat; Tridecylneopentanoat.

11. Zusammensetzung nach Anspruch 10, **dadurch gekennzeichnet, dass** der Ester unter Isopropylpalmitat, 2-Ethylhexylpalmitat, Isopropylmyristat, tert-Butylmyristat, Butylisostearat, Isobutylstearat, Isononylisononanoat und Isostearylneopentanoat ausgewählt ist.

12. Zusammensetzung nach Anspruch 11, **dadurch gekennzeichnet, dass** der Ester unter Isopropylpalmitat, Isopropylmyristat, tert-Butylmyristat, Isononylisononanoat und Isostearylneopentanoat ausgewählt ist.

13. Zusammensetzung nach einem der Ansprüche 1 bis 12, **dadurch gekennzeichnet, dass** die Ester in Konzentrationen von 0,5 bis 5 Gew.-%, bezogen auf das Gesamtgewicht der Zusammensetzung, und insbesondere 0,8 bis 3 Gew.-% enthalten sind.

14. Zusammensetzung nach einem der Ansprüche 1 bis 13, **dadurch gekennzeichnet, dass** die Zusammensetzung ferner mindestens ein kationisches Polymer enthält.

15. Zusammensetzung nach Anspruch 14, **dadurch gekennzeichnet, dass** das kationische Polymer unter den quartären Celluloseetherderivaten, Homopolymeren von Diallyldimethylammoniumsalzen, Copolymeren von Diallyldimethylammoniumsalzen und Acrylamid, kationischen Polysacchariden, quartären Copolymeren von Vinylpyrrolidon und einem Vinylimidazolsalz ausgewählt ist.

16. Zusammensetzung nach Anspruch 15, **dadurch gekennzeichnet, dass** das kationische Polymer unter den Polymeren ausgewählt ist, die aus Wiederholungseinheiten der folgenden Formel aufgebaut sind: 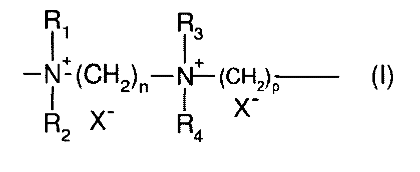 in der die Gruppen R₁, R₂, R₃ und R₄, die gleich oder verschieden sind, eine Alkyl- oder Hydroxyalkylgruppe mit etwa 1 bis 4 Kohlenstoffatomen bedeuten, n und p ganze Zahlen im Bereich von etwa 2 bis 20 sind und X- ein Anion bedeutet, das von einer anorganischen oder organischen Säure abgeleitet ist.

17. Zusammensetzung nach einem der Ansprüche 15 bis 16, **dadurch gekennzeichnet, dass** das kationische Polymer 0,001 bis 10 Gew.-%, vorzugsweise 0,005 bis 5 Gew.-% und noch bevorzugter 0,01 bis 3 Gew.-% des Gesamtgewichts der fertigen Zusammensetzung ausmacht.

18. Zusammensetzung nach einem der Ansprüche 1 bis 16, **dadurch gekennzeichnet, dass** das kosmetisch akzeptable wässrige Medium ausschließlich aus Wasser oder aus einem Gemisch von Wasser und einem kosmetisch akzeptablen Lösungsmittel besteht.

19. Zusammensetzung nach Anspruch 18, **dadurch gekennzeichnet, dass** das Lösungsmittel unter den niederen Alkoholen mit 1 bis 4 Kohlenstoffatomen, wie Ethanol, Isopropanol, tert-Butanol, n-Butanol; Alkylenglycolen, wie Propylenglycol oder Hexylenglycol, und Glycerin ausgewählt ist.

20. Zusammensetzung nach einem der Ansprüche 1 bis 19, **dadurch gekennzeichnet, dass** sie ferner einen oder mehrere Zusatzstoffe enthält, die unter den kationischen grenzflächenaktiven Stoffen, anionischen, nichtionischen oder amphoteren Polymeren, Proteinen, Proteinhydrolysaten, Ceramiden, Pseudoceramiden, pflanzlichen Ölen, Fettsäuren, Hydroxysäuren, Vitaminen, Provitaminen wie Panthenol, Siliconen, die flüchtig oder nicht flüchtig und in dem Medium löslich oder unlöslich sind, UV-Filtern, Hydratisierungsmitteln, Antischuppenmitteln, Wirkstoffen gegen Seborrhoe, Wirkstoffen gegen Haarausfall, Radikalfängern für freie Radikale, Trübungsmitteln und deren Gemischen ausgewählt sind.

21. Verwendung einer Zusammensetzung nach einem der Ansprüche 1 bis 20 für die Reinigung und/oder zum Abschminken von Keratinsubstanzen.

22. Verfahren für die Reinigung und Pflege von Keratinsubstanzen, wie Haaren, das darin besteht, eine wirksame Menge einer Zusammensetzung nach einem der Ansprüche 1 bis 20 auf die feuchten Keratinsubstanzen aufzutragen und anschließend nach einer gegebenenfalls abgewarteten Einwirkzeit mit Wasser zu spülen.
